(19) Europäisches Patentamt
European Patent Office
Office européen des brevets

(11) Veröffentlichungsnummer: **0 301 371 B1**

(12) # EUROPÄISCHE PATENTSCHRIFT

(45) Veröffentlichungstag der Patentschrift: **12.12.90**

(51) Int. Cl.⁵: **C07D 307/79**, C07D 307/86, C07D 405/12, A61K 31/34

(21) Anmeldenummer: **88111571.1**

(22) Anmeldetag: **19.07.88**

(54) **Basisch substituierte Benzofurancarbonsäureamide, Verfahren zu ihrer Herstellung und sie enthaltende therapeutische Mittel.**

(30) Priorität: **25.07.87 DE 3724756**

(43) Veröffentlichungstag der Anmeldung: **01.02.89 Patentblatt 89/5**

(45) Bekanntmachung des Hinweises auf die Patenterteilung: **12.12.90 Patentblatt 90/50**

(84) Benannte Vertragsstaaten: **AT BE CH DE ES FR GB GR IT LI NL SE**

(56) Entgegenhaltungen:
**EP-A- 0 147 044**
**FR-A- 2 578 539**

**ARZNEIMITTEL FORSCHUNG, Band 21, Nr. 7, Juli 1971, Seiten 1026-1028, Aulendorf, DE; O.H. HISHMAT et al.: "Sythesis of some benzofuran-carboxamide derivatives and their analgesic activty"**

(73) Patentinhaber: **BASF Aktiengesellschaft, Carl-Bosch-Strasse 38, D-6700 Ludwigshafen(DE)**

(72) Erfinder: **Schlecker, Rainer, Dr., Suedring 8, D-6719 Bissersheim(DE)**
Erfinder: **Teschendorf, Hans-Jürgen, Dr., Georg-Nuss-Strasse 5, D-6724 Dudenhofen(DE)**
Erfinder: **Unger, Liliane, Dr., Waltraudenstrasse 14, D-6700 Ludwigshafen(DE)**

EP 0 301 371 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue, basisch substituierte Benzofurancarbonsäureamide, Verfahren zu ihrer Herstellung und sie enthaltende therapeutische Mittel, vor allem zur Therapie einer Reihe von Störungen des Zentralnervensystems, exogener psychischer Störungen und von Dyskinesien.

In EP-A 147 044 werden basisch substituierte 2,3-Dihydrobenzo(b)-furan-7-carbonsäureamide, die frei von phenolischen Hydroxylgruppen sind, mit antiemetischer und/oder antipsychotischer Wirkung beschrieben.

2-Methoxysubstituierte Benzamide mit basischer Seitenkette sind als antidopaminerg wirkende Arzneimittel in der Literatur beschrieben (Bernd Testa, J. Med. Chem. 26 (1983), 203–207). Die bekannteste Verbindung ist Sulpirid, dessen orale Verfügbarkeit jedoch nicht befriedigt.

Es wurde nun gefunden, daß Verbindungen der allgemeinen Formel I in ihrer zentralen antidopaminergen Wirkung Sulpirid deutlich übertreffen. Diese hohe zentrale Verfügbarkeit ist angesichts des für peripher wirkende Verbindungen [B. Pourrias, Arch. int. Pharmacodyn. 274, 223 (1985)] typischen Aromatensubstitutionsmusters überraschend.

In der allgemeinen Formel I bedeuten

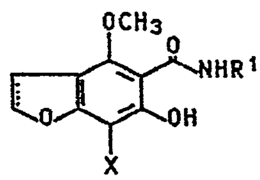

I

X ein Wasserstoffatom oder eine Methoxygruppe,
$R^1$ einen Rest der allgemeinen Formel

$$-CHR^2-CHR^3-(CR^4R^5)_n-NR^6R^7 \qquad II,$$

worin $R^2$ bis $R^5$ unabhängig voneinander je ein Wasserstoffatom oder eine Alkylgruppe mit 1 bis 6 Kohlenstoffatomen, $R^6$ und $R^7$ unabhängig voneinander einen gegebenenfalls verzweigten Alkylrest mit 1 bis 6 Kohlenstoffatomen oder einen gegebenenfalls am Kern methyl- oder halogen-substituierten Benzylrest darstellen und jeweils einer oder zwei der Reste $R^2$ bis $R^6$ zusammen mit $R^7$ eine Alkylenkette mit 2 bis 6 Kohlenstoffatomen bilden können, die durch einen Ethersauerstoff unterbrochen sein kann;

n steht für 0 oder 1.

Die Doppelbindung 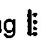 im Furanring kann hydriert sein.

Aus der allgemeinen Formel II ergeben sich also folgende allgemeine Strukturmöglichkeiten für die Gruppe -HN-$R^1$:

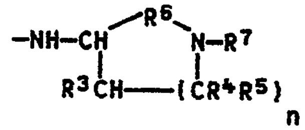

IIa

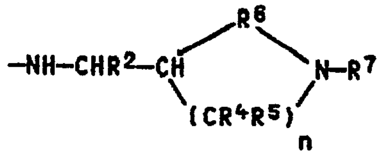

IIb

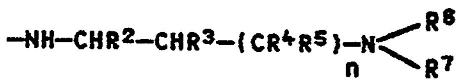

IIc

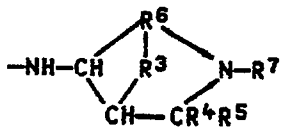

IId

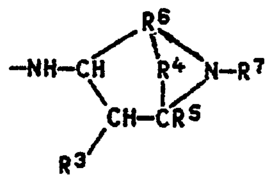

IIe

Beispiele sind:

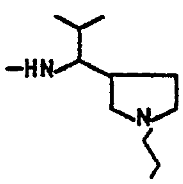
,
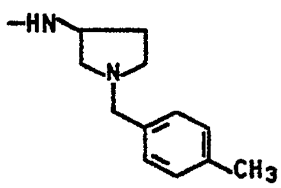
,
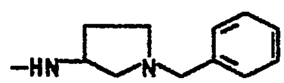

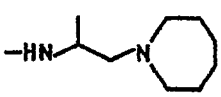
,
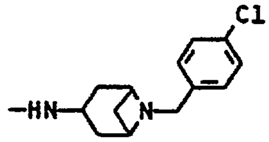
,
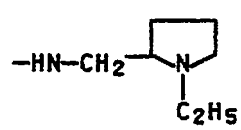

Die Verbindungen der allgemeinen Formel I werden hergestellt durch Umsetzung eines reaktiven Säurederivates III mit einem Amin $H_2N$-$R^1$ nach allgemein bekannten Methoden.

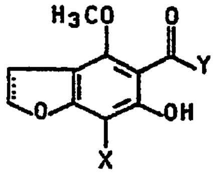

III

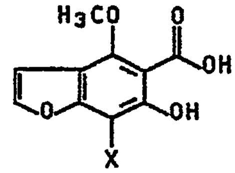

IV

Y stellt eine Austrittsgruppe dar, die sich durch Nukleophile verdrängen läßt, beispielsweise ein Chlor- oder Bromatom, eine Methoxy- oder Ethoxygruppe, einen Oxysuccinimid-, 1-Imidazolyl- oder Ethoxycar-

bonyloxyrest. Die Herstellung dieser Säurederivate aus der Säure IV sowie deren Umsetzung mit Aminen ist literaturbekannt und ist beispielsweise in Houben-Weyl, Methoden der Org. Chemie, 4. Auflage, E5, S. 941 - 991 beschrieben.

Die Säure IV wird in einfacher Weise durch oxydative Ringöffnung der natürlichen Furochromone Khellin Va und Visnagin Vb hergestellt (JACS 75, 4992 (1953)).

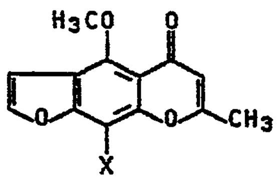

X = $OCH_3$ Va

X = H Vb

Hierdurch sind die Verbindungen der allgemeinen Formel I deutlich leichter zugänglich als andere antidopaminerge Benzamide, bei denen geeignete Substituenten synthetisch in den Benzolring eingeführt werden müssen (J. Med. Chem. 21, 61 (1986), 25, 1280 (1982)).

Verbindungen der allgemeinen Formel I, in denen der Furanring hydriert ist, lassen sich ausgehend von der Säure

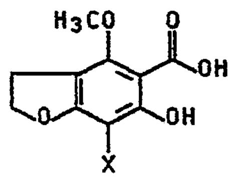

VI

in analoger Weise erhalten. Diese kann beispielsweise hergestellt werden durch Hydrierung der Säure IV oder eines ihrer Salze nach an sich bekannten Methoden.

Einige der erfindungsgemäßen Verbindungen I besitzen ein Chiralitätszentrum und werden als Racemate erhalten, die durch bekannte Methoden, beispielsweise durch Bildung diastereomerer Salze mit optisch aktiven Hilfssäuren, in die Enantiomeren getrennt erhalten werden können. Weiterhin können die optisch reinen Verbindungen I durch Verwendung des enantiomerenreinen Amins $H_2N{-}R^1$ erhalten werden.

Die erfindungsgemäßen Verbindungen eignen sich zur Therapie einer Reihe von Störungen des Zentralnervensystems, insbesondere zur Behandlung psychotischer Erkrankungen wie der Schizophrenie, sowie zur Behandlung exogener psychischer Störungen, insbesondere solcher, die mit einer Antriebsminderung einhergehen. Weiterhin können die Substanzen bei der Behandlung von Dyskinesien (z.B Chorea Huntington, tardive Dyskinesien nach Neuroleptikatherapie) eingesetzt werden.

Weitere Anwendungsgebiete sind die Therapie von Erbrechen, von Ulcus ventriculi od. Ulcus duodeni sowie von Motilitätsstörungen des Magen- und Darmtrakts.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise, beispielsweise durch Vermischen des Wirkstoffes mit den an sich in solchen Präparaten üblichen festen und flüssigen Träger- und Hilfsstoffen.

Die Mittel können peroral, parentral oder topisch verabreicht werden. Derartige Zubereitungen sind beispielsweise Tabletten, Filmtabletten, Dragees, Kapseln, Pillen, Pulver, Lösungen oder Suspension, Infusions- oder Injektionslösungen.

Die therapeutischen Mittel können die erfindungsgemäß zu verwendenden Verbindungen in einer Einzeldosis von 0,03 bis 3 mg pro kg-Gew., also 2 bis 200 mg pro Tablette, enthalten und täglich in einer oder mehreren Dosierungen je nach Art und Schwere der Erkrankungen verabreicht werden.

Üblicherweise verwendete pharmazeutisch technische Hilfsstoffe sind beispielsweise für die lokale Anwendung Alkohole, wie Ethanol, Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol, Polyethylenglykol-Stearat sowie ethoxylierter Fettalkohol, für die systemische Anwendung Milchzucker, Porpylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Bleichmittel usw. zugesetzt werden. Voraussetzung ist, daß alle bei der Herstellung pharmazeutischer Zubereitungen verwendeten Stoffe toxikologisch unbedenklich und mit den verwendeten Wirkstoffen verträglich sind.

Herstellung von 2,3-Dihydro-6-hydroxy-4,7-dimethoxybenzofuran-5-carbonsäure (als Vorprodukt)

5 g 6-Hydroxy-4,7-dimethoxybenzofuran-5-carbonsäure werden in 75 ml Ethanol/25 ml$H_2O$/0,8 g NaOH in Anwesenheit von 1 g Pd/C (10 %) bei Normaldruck und 50°C bis zur Beendigung der Wasserstoffaufnahme hydriert. Die Mischung wird filtriert, das Ethanol abdestilliert, die Wasserphase angesäuert und der Feststoff abgesaugt. Man erhält 3,7 g Produkt, das ohne Reinigung weiter umgesetzt wird.

6-Hydroxy-4,7-dimethoxybenzofuran-5-carbonsäure-(N-hydroxy-succinimid)-ester (als Vorprodukt)

Zu 24,3 g 6-Hydroxy-4,7-dimethoxybenzofuran-5-carbonsäure und 11,7 g N-Hydroxysuccinimid in 300 ml $CH_2Cl_2$ wird bei Raumtemperatur eine Lösung von 21,0 g Dicyclohexylcarbodiimid in $CH_2Cl_2$ zugetropft. Die Mischung wird 3 Stdn. bei Raumtemperatur gerührt, filtriert, das Filtrat mit Wasser gewaschen und das Lösungsmittel abgezogen. Nach Umkristallisation des Rückstandes aus Isopropanol erhält man 20,7 g Produkt, Fp. 136°C.

In analoger Weise werden beispielsweise folgende Vorprodukte hergestellt:
6-Hydroxy-4-methoxybenzofuran-5-carbonsäure-(N-hydroxysuccinimid)ester, Fp. 134°C.
2,3-Dihydro-6-hydroxy-4,7-dimethoxybenzofuran-5-carbonsäure-(N-hydroxy-succinimid)ester, Fp. 133°C.

Beispiel 1

N-[(1-Ethyl-2-pyrrolidinyl)methyl]-6-hydroxy-4,7-dimethoxybenzofuran-5-carbonsäureamid.

Zu einer Lösung von 10 g 6-Hydroxy-4,7-dimethoxybenzofuran-5-carbonsäure-(N-hydroxysuccinimid)ester in 100 ml $CH_2Cl_2$ wurden unter Kühlung 4,7 g 1-Ethyl-2-(aminomethyl)pyrrolidin getropft. Die Mischung wurde über Nacht bei Raumtemperatur gerührt, anschließend mit $NaHCO_3$-Lösung und Wasser gewaschen. Die organische Phase wurde abgetrennt, mit $Na_2SO_4$ getrocknet und das Lösungsmittel abdestilliert. Der Rückstand wurde aus Ethanol umkristallisiert. Man erhielt 7,0 g, Fp. 86°C.

In analoger Weise wurden, ausgehend von den entsprechenden Säure- und Aminkomponenten, die nachstehenden erfindungsgemäßen Verbindungen hergestellt.

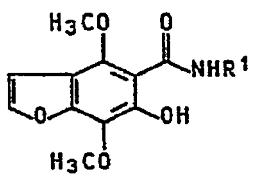

| Bsp.Nr. | $-HN-R^1$ | Fp. [°C] |
|---|---|---|
| 2 | 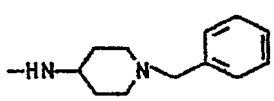 | 212 (Hydrochlorid) |
| 3 | 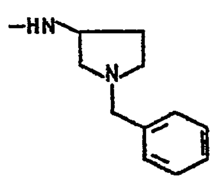 | 198 (Hydrochlorid) |
| 4 | 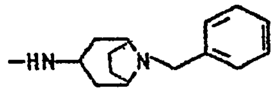 | 138 |
| 5 | 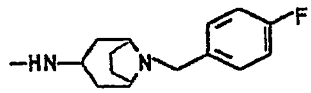 | 128 |

| Bsp.Nr. | —HN—R¹ | Fp. [°C] |
|---|---|---|
| 6 | 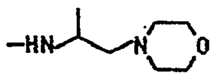 | 121 |
| 7 | 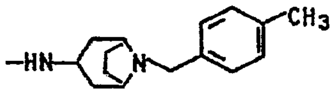 | 151 |
| 8 | 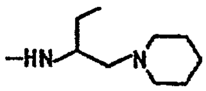 | Öl |
| 9 | 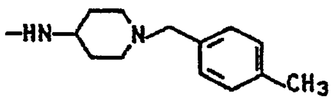 | 217 (Hydrochlorid) |
| 10 | 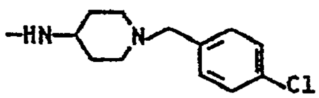 | 172 (Hydrochlorid) |
| 11 | 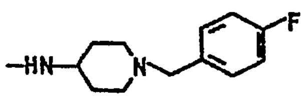 | 205 (Hydrochlorid) |

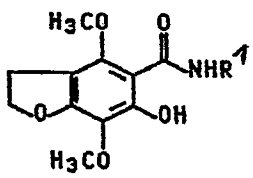

| Bsp.Nr. | —HN—R¹ | Fp. [°C] |
|---|---|---|
| 12 | 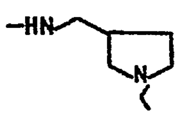 | 189 (Hydrochlorid) |
| 13 | 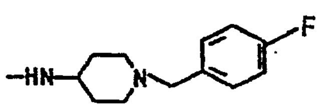 | 204 (Hydrochlorid) |

| Bsp.Nr. | $-HN-R^1$ | Fp. [°C] |
|---|---|---|
| 14 | 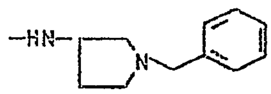 | 82 |
| 15 | 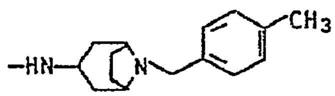 | 127 |
| 16 | 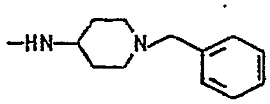 | 249 (Hydrochlorid) |

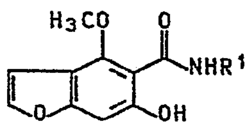

| Bsp.Nr. | $-HN-R^1$ | Fp. [°C] |
|---|---|---|
| 17 | 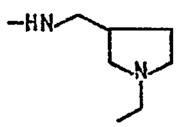 | 153 (Hydrochlorid) |
| 18 | 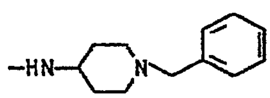 | 151 (Hydrochlorid) |

## Patentansprüche

1. Basisch substituierte Benzofurancarbonsäureamide der Formel

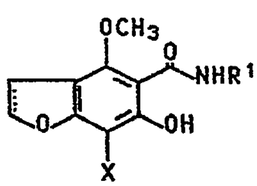

I

wobei X ein Wasserstoffatom oder eine Methoxygruppe und $R^1$ einen Rest der allgemeinen Formel

$$-CHR^2-CHR^3-(CR^4R^5)_n-NR^6R^7 \qquad II,$$

bedeutet, in der $R^2$ bis $R^5$ unabhängig voneinander ein Wasserstoffatom oder eine Alkylgruppe mit 1 bis 6 Kohlenstoffatomen und $R^6$ und $R^7$ unabhängig voneinander einen verzweigten oder unverzweigten Alkylrest mit 1 bis 6 Kohlenstoffatomen oder einen gegebenenfalls am Kern methyl- oder halogensubstituierten Benzylrest darstellen und jeweils einer oder zwei der Reste $R^2$ bis $R^6$ zusammen mit $R^7$ eine Alkylenkette mit 2 bis 6 Kohlenstoffatomen bilden können, die durch einen Ethersauerstoff unterbrochen sein kann,
n 0 oder 1 und

⁞ eine Ein- oder Zweifachbindung bedeutet.

2. Verfahren zur Herstellung von basisch substituierten Benzofurancarbonsäureamiden nach Anspruch 1 durch Umsetzung eines reaktiven Säurederivates der Formel III, in der X die in Anspruch 1 genannte Bedeutung hat und Y eine Austrittsgruppe darstellt, mit einem Amin $H_2N-R^1$ nach an sich bekannten Methoden.

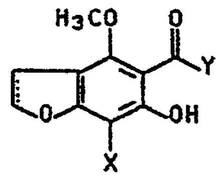

III

3. Verfahren zur Herstellung des Säurederivates III von Anspruch 2 aus der durch oxidative Ringöffnung der natürlichen Furochrome Khellin (Va) und Visnagin (Vb)

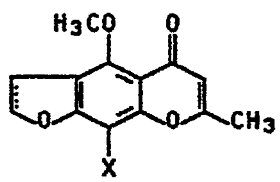

X = $OCH_3$ Va

X = H Vb

erhaltenen Säure der Formel IV

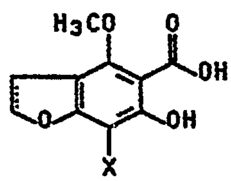

IV

durch Umsetzung zum Säurederivat III nach an sich bekannten Methoden.

4. Therapeutisches Mittel, das als Wirkstoff 2 bis 200 mg eines basisch substituierten Benzofurancarbonsäureamids nach Anspruch 1 enthält.

## Revendications

1. Amide d'acide benzofurancarboxylique substitué basiquement, de formule

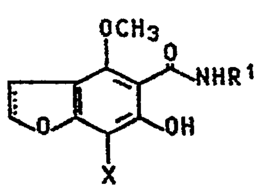

I

x signifiant un atome d'hydrogène ou un groupe méthoxy et
$R^1$, un reste de la formule générale

$$-CHR^2-CHR^3-(CR^4R^5)_n-NR^6R^7 \qquad II,$$

dans laquelle
$R^2$ à $R^5$ représentent, indépendamment l'un de l'autre, un reste alkyle, ramifié ou non ramifié, ayant 1 à 6 atomes de carbone ou un reste benzyle éventuellement substitué sur le noyau par méthyle ou halogène

et un ou deux des restes $R^2$ à $R^6$ peuvent respectivement former, avec $R^7$, une chaîne alkyle ayant 2 à 6 atomes de carbone, qui peut être interrompue par un oxygène d'éther
n représente 0 ou 1 et

⁞ représente une liaison simple ou double.

2. Procédé de préparation d'amide d'acide benzofurancarboxylique substitué basiquement selon la revendication 1 par réaction d'un dérivé d'acide, réactif, de la formule III, dans laquelle X a la signification donnée dans la revendication 1 et Y représente un groupe éliminable, avec une amine $H_2N-R^1$ selon des méthodes connues en soi

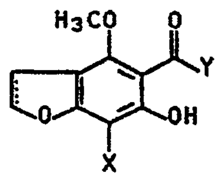

III

3. Procédé de préparation du dérivé d'acide III de la revendication 2, à partir de l'acide de formule IV

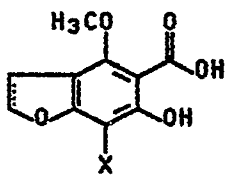

IV

obtenu par ouverture du noyau, par voie oxydante, des furochromes naturels, khelline (Va) et visnagine (Vb)

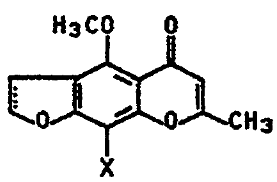

$X = OCH_3$ Va

$X = H$ Vb

par réaction conduisant au dérivé d'acide III selon des méthodes connues en soi.

4. Agent thérapeutique qui contient, comme principe actif, 2 à 300 mg d'un amide d'acide benzofurancarboxylique substitué basiquement selon la revendication 1.

## Claims

1. A benzofurancarboxamide having a basic substituent, of the formula

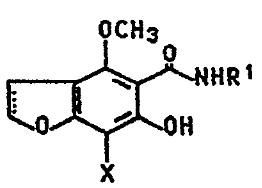

I

where
X is hydrogen or methoxy,
$R^1$ is a radical of the formula

$$-CHR^2-CHR^3-(CR^4R^5)_n-NR^6R^7 \qquad II,$$

where $R^2$ to $R^5$ are each, independently of one another, hydrogen or alkyl of 1 to 6 carbon atoms, $R^6$ and $R^7$ are, independently of one another, a branched or unbranched alkyl of 1 to 6 carbon atoms, or benzyl

which may be methyl- or halogen-substituted on the nucleus, and, in each case, one or two of $R^2$ to $R^6$ can form together with $R^7$ an alkylene chain of 2 to 6 carbon atoms which can be interrupted by an ether oxygen;
n is 0 or 1, and

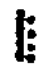 is a single or double bond.

2. A process for the preparation of a benzofurancarboxamide having a basic substituent, as claimed in claim 1, by reacting a reactive acid derivative of the formula III in which X has the meaning stated in claim 1, and Y is a leaving group, with an amine $H_2N{-}R^1$ by conventional methods.

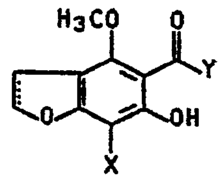

III

3. A process for the preparation of the acid derivative III of claim 2 from the acid of the formula IV

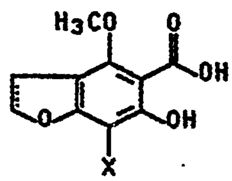

IV

obtained by oxidative ring opening of the natural furochromones khellin (Va) and visnagin (Vb):

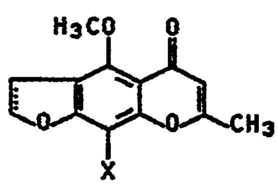

by conversion into the acid derivative III by conventional methods.

4. A therapeutic agent which contains as active compound 2 to 200 mg of a benzofurancarboxamide having a basic substituent as claimed in claim 1.